# EUROPEAN PATENT APPLICATION

(11) **EP 3 910 332 A1**
(43) Date of publication of application: **17.11.2021**
(21) Application number: 21173190.6
(22) Date of filing: 11.05.2021
(51) Int. Cl.: G01N 33/50, G01N 33/569

(54) **HOME TEST FOR THE INTEGRATED DIAGNOSIS OF SARS-COV-2 INFECTION**

(30) Priority: 15.05.2020 IT 202000011182
(71) Applicant: Molipharma R&D S.r.l., 86100 Campobasso (CB) (IT)
(72) Inventor: PANI, Giovanbattista, Rome (IT); BARTOCCIONI, Emanuela, Rome (IT); POSTERARO, Brunella, Rome (IT); SANGUINETTI, Maurizio, Rome (IT)
(74) Representative: Di Giovine, Paolo

(57) **Abstract**

The present invention relates to a method for the integrated diagnosis of SARS-CoV-2 infection, based upon the detection of SARS-CoV-2 viral particles and/or anti-SARS-CoV-2 IgA-class immunoglobulins in a saliva sample. The invention further relates to a kit usable at home for the integrated diagnosis of SARS-CoV-2 infection.

## Description

### FIELD OF THE INVENTION

The present invention relates to a method for the integrated diagnosis of SARS-CoV-2 infection, based upon the detection of SARS-CoV-2 viral particles and/or anti-SARS-CoV-2 IgA-class immunoglobulins in a saliva sample. The invention further relates to kits usable at home for the integrated diagnosis of SARS-CoV-2 infection.

### STATE OF ART

The developments of Covid-19 epidemic have dramatically shown an insufficient capability of most National Health Systems in diagnosing the SARS-CoV-2 virus infection not only in large asymptomatic or paucisymptomatic population groups, but unfortunately even in the individuals with strong clinical suspect of having contracted the disease and which are confined at home and not at the hospital. In this scenario, the current diagnostic capabilities appear on a global scale strongly undersized. Notwithstanding several progresses have been performed in speeding-up and simplifying virological tests of molecular type (PCR) based upon Real-Time amplification of the patient's nasal/oropharyngeal swab, such method, the only one currently accredited for the virological diagnosis of SARS-CoV-2 infection, has strong accessibility limits, by requiring the intervention of a professional operator for sampling, as well as the sample transfer to a specialized labour. Such method further involves a specific infectious disease risk for the operators themselves.

The so-called, currently more and more widespread, "quick tests", overcome only partially the above-described limits. These tests strongly identify with serological tests for the qualitative detection of specific anti-CoV-2 IgG/IgM-class antibodies, in immunochromatography (Lateral Flow Assay). The test can be easily performed by using a drop of blood or serum, but the use is however reserved, at the moment, exclusively to the hospital or to an outpatient department. Whereas some studies have questioned the diagnostic utility of this type of test in the infection initial phases (Cassaniti I. et al. J. Med. Virol. 2020), they are certainly capable of providing information about the occurred contact with the virus after a sufficient number of days (Guo L. et al. Clin. Infect. Dis. 2020), even if there are still doubts on the real protective meaning of such antibody response.

Surprisingly, most above-mentioned "quick" tests do not seem to include the evaluation of IgA-class immunoglobulins, notoriously fundamental in mucosal immunity and more specifically in the protection against infections by respiratory viruses, including coronavirus (Allie, S. R. & Randall, T. D. Clinic Science 2017; Callow, K. A. J. Hyg. (Lond). 1985). The reason of such choice probably lies in the fact that the specific IgA immunoglobulins are less concentrated in the serum than the immunoglobulins of other classes. However, they are the most represented Ig in the nasal secretions. Moreover, studies about mucosal immunity against flu viruses have shown that the slgA appear early in the nasal secretions after the first infection (3-5 days) and here remain for 3-5 months, by playing a primary role in the resistance to re-infection (Gianchecchi, E. et al. Viruses 2019).

From the combination of these pieces of information it is clear that the detection of secretory IgA against SARS-CoV-2 could represent a precious and early sign of individual immunity against this agent.

Among the several commercial serological tests currently available for SARS-CoV-2, a list recently published by Johns Hopkins University shows only a conventional ELISA test specific for the detection of IgA (Euroimmun, Germany).

In front of this scenario, the need remains evident for developing new methods and commercial kits providing a possibility of quick and reliable diagnosis of SARS-CoV-2 infections and which could be used at home by not-expert personnel.

### SUMMARY OF THE INVENTION

The purpose of the present invention is to obviate the limitations of the currently available methods for diagnosing SARS-CoV-2 infections, by providing a simple diagnostic test, having low technological impact, and which can be used at home, based upon the integrated detection (a) of SARS-CoV-2 virus (antigen), and b) of specific secretory (anti SARS-CoV-2) IgA-class immunoglobulins, in a sample of sputum or saliva from posterior oropharynx.

The integrated detection of SARS-CoV-2 virus and of IgA-immunoglobulins in saliva sample gives the method of the present invention a high reliability and good sensitivity for the infection diagnosis. Advantageously, the execution of the test, the invention relates to, does not further require the intervention of a sanitary operator, then it can be made directly by not-expert personnel at home and interpreted by the physician, for example, by means of simple photographic documents.

The use of the at-home testing as described in the present invention wants to represent only a first level of evaluation of the infective and immunological state of the individual, but accessible in a capillary manner and without any risk for the operators, with evident advantages not only for managing the current epidemic emergency, but even for monitoring over time its evolution and for epidemiological studies and interventions of public health on large scale.

The possibility of reaching in a capillary way symptomatic or asymptomatic individuals for a sufficiently sensitive and specific evaluation both of the presence of infection and of the development of a presumably protective immunity, could in fact not only address in a targeted way the route of the single individual (with possible isolation or hospitalization measures), but could be a precious source of epidemiological information for monitoring the epidemic emergency and for its sanitary and political management.

Therefore, a first aspect of the present invention relates to a method for diagnosing SARS-CoV-2 infection based upon the detection in a biological sample of SARS-CoV-2 viral particles and/or anti-SARS-CoV-2 IgA-class immunoglobulins, wherein said biological sample is a saliva sample.

A second aspect of the present invention relates to a kit for diagnosing SARS-CoV-2 infection comprising one or more means for the detection, by agglutination tests, of SARS-CoV-2 viral particles in a saliva sample.

A third aspect of the present invention relates to a kit for diagnosing SARS-CoV-2 infection comprising one or more means for the detection, by lateral flow immunochromatography test (LFA), of SARS-CoV-2 viral particles in a saliva sample. A fourth aspect of the invention relates to a kit for diagnosing SARS-CoV-2 infection comprising one or more means for the detection, by agglutination tests, of anti-SARS-CoV-2 IgA-class immunoglobulins in a saliva sample.

At last, a fifth aspect of the present invention relates to a kit for diagnosing SARS-CoV-2 infection, comprising one or more means for the detection, by lateral flow immunochromatography test (LFA), of anti-SARS-CoV-2 IgA-class immunoglobulins in a saliva sample.

Other advantages and features of the present invention will result evident from the following detailed description.

### BRIEF DESCRIPTION OF THE DRAWINGS

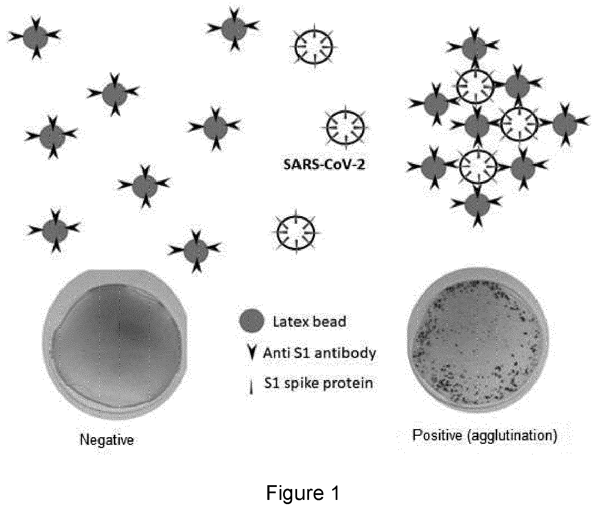

Figure 1. Schematic illustration of the components and of the operating principle of the method according to an embodiment of the present invention. Latex beads CoV2-Agglutination Test. The suspension of latex beads has a diffused milky aspect in absence of antigen (virus) whereas it forms aggregates visible to naked eye in presence thereof. The two shown examples of negative and positive reaction have only an explanatory value and they have not been obtained with this specific test.

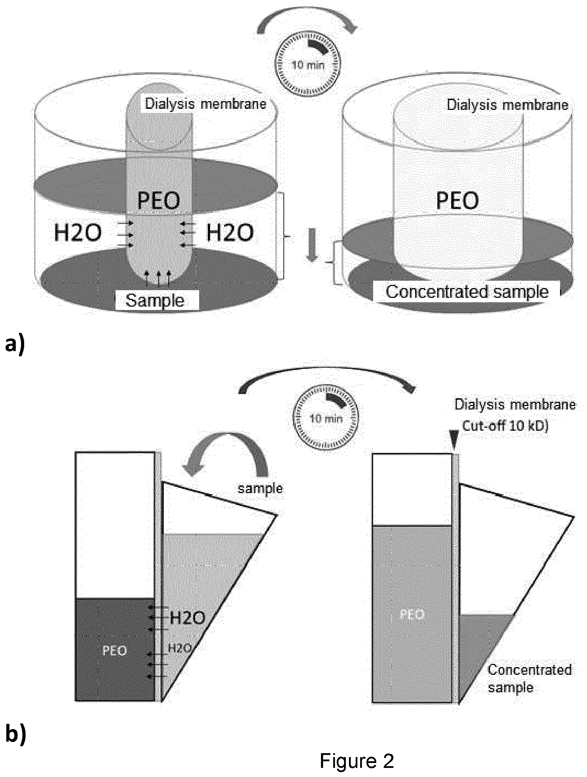

Figure 2. Two possible formats for a salivary fluid concentrator simple to be used and compatible with a use at home to be used in the methods and kits according to a preferred embodiment of the present invention. The format a) is represented by a sachet to be dipped into the sample. In the format b) the concentrator comprises two chambers, one for PEO and the other one for the sample. In both cases the concentration take place by direct osmosis ("forward osmosis") by drawing water by part of a concentrated solution ("draw solution") of Polyethylene Oxide (PEO) having high molecular weight (>200 kD). The interposition of a dialysis membrane with cut-off equal to 10-12 kD between the two solutions allows to concentrate in the saliva all components with larger sizes, including micro-organisms and antibodies.

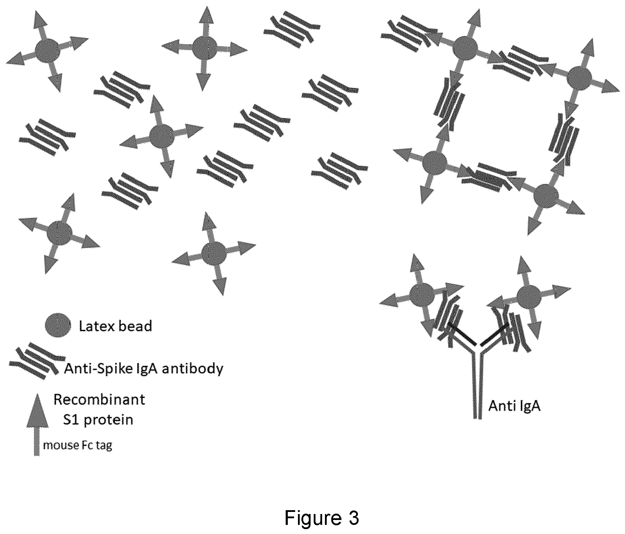

Figure 3. Schematic illustration of the method according to an embodiment of the present invention. Test for highlighting anti SARS-CoV-2 (Spike protein) salivary IgA by means of latex beads Agglutination Test. The principle is similar to the one described in figure 1, but in this case the viral antigen is adsorbed on the surface of the beads, whereas the agglutinating agent (under the form of specific slgA) is contained in the sample. The test components are illustrated by the legend. It is to be noted that the dimeric nature of slgA eases the agglutination process. The structure of the recombinant antigen is highlighted too, wherein the viral component (S1) is fused to the Fc portion of murine IgG. The agglutinating power of salivary IgA could further be increased by anti IgA antibodies (mono or polyclonal antibodies, orange element in figure), through the aggregation of slgA bound to different beads.

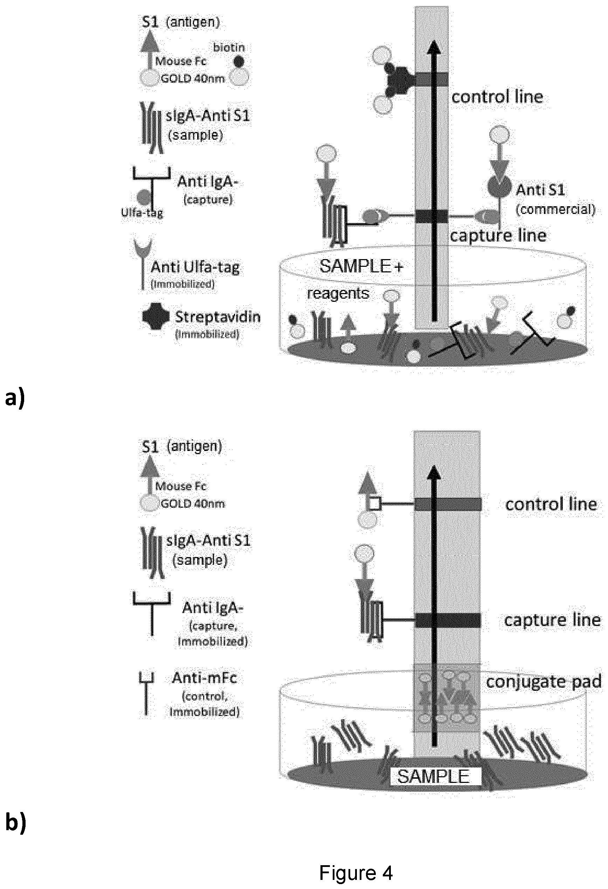

Figure 4. Schematic illustration of the method according to an embodiment of the present invention. Immunochromatographic test (Lateral Flow Assay) for highlighting anti CoV2-SP1 slgA in the sputum/saliva of proband. The different test components are illustrated in the legend. In the configuration illustrated in a) the capture line includes an anti Ulfa-tag reagent. On the left side, the immunocomplex SP1-gold/slgA is blocked on the capture line by an anti IgA antibody labelled with Ulfa-tag (positive result). On the right side, an anti SP1 commercial reagent, labelled too with Ulfa-tag, can be used as an additional positive control. The control line signal, which includes Streptavidin, is provided by Gold particles labelled with biotin.
   b) The test can even be imagined in an ad hoc configuration (which requires industrial manufacture), with the anti IgA reagent directly immobilized on the capture line, and an anti-mouse antibody Fc (mFc) on the control line. In this second configuration it is even possible to imagine that the SP1-gold conjugate is already present, in a dried form, on the small strip. In both configurations the test is imagined as a "dipstick" which the proband dips into a container containing saliva or the concentration thereof.

### DETAILED DESCRIPTION

### GLOSSARY

The terms used in the present description are as generally understood by the person skilled in the art, except where differently designated.
With the acronym SARS-CoV-2, deriving from English "Severe Acute Respiratory Syndrome - Coronavirus - 2), the present invention relates to the coronavirus 2 due to severe acute respiratory syndrome, a viral strain of the SARS-related coronavirus (or SARS-CoV) species, belonging to the genus Betacoronavirus, discovered around end of 2019. In particular it relates to the seventh coronavirus recognized able to infect human beings. The most external layer of the SARS-CoV-2 virus is characterized by glycoproteins S (or "Spike", from English "tip", "spike") assembled to form trimeric structures constituting the characteristic "crown" which surrounds the virion. The glycoproteins S determine the virus specificity for the epithelial cells of the respiratory tract. The ectodomain of the Spike proteins is characterized by two distinct domains: a N-terminal domain, called S1, responsible for the bond to the human receptor ACE2 in the epithelial cells, and a C-terminal domain, called S2, responsible for fusion.
A second coating protein, known as hemagglutinin esterase dimer (HE), smaller than protein S, performs an important function during the phase of releasing the virus inside the host cells. The virus also consists of membrane proteins crossing the external coating, by interacting inside the virion with the RNA-protein complex.
The virus genome consists of one single filament of RNA having positive polarity with a great size (from 27 to 32 kb in the different viruses), associated to the N protein, which increases the stability thereof.
With the acronym COVID-19 (from English acronym CoronaVIrus Disease 19), or acute respiratory disease by SARS-CoV-2, a respiratory infective disease is meant caused by SARS-CoV-2 virus. The virus transmits by air, much often under the form of respiratory small drops. The disease is characterized by a series of symptoms described as flu like, thereamong fever, cough, shortness of breath and gastrointestinal disorders. In the most serious cases a pneumonia, a syndrome by acute respiratory distress, sepsis, septic shock can take place, until causing the patient's death.
With the acronym IgA, the present invention relates to A-class immunoglobulins. IgA constitute about 15% of immunoglobulins existing in blood but they are present even in saliva, in tears, in the gastric secretions, and in the breast milk. Then, IgA can provide a protection against infections of mucosae, including the respiratory tract, high and low tracts and gastrointestinal tract, stomach and intestine. With the acronym slgA the invention specifically relates to A-class secretory immunoglobulins, mainly existing in the nasal and mucosal secretions in mainly dimeric form, but even tri-or tetrameric form. They include, with respect to the plasmatic IgA, two additional protein components, chain J ("joining") and Secretory component. slgA are secreted by the plasma cells adjacent to the mucosal epithelial cells and represent the main humoral mediator of the mucosal immunity.

As mentioned, the object of the present invention is to provide a quick and reliable test, and which can be made at home, for diagnosing SARS-CoV-2 infections. The test of the present invention is based upon the integrated detection of the SARS-CoV-2 virus, and/or of the anti-SARS-CoV-2 secretory IgA-class immunoglobulins, antibody agents immediately involved in the defence from contagion by the virus and particularly important for evaluating the mucosal immunity.
Therefore, a first aspect of the present invention relates to a method for diagnosing SARS-CoV-2 infection based upon the detection in a biological sample of SARS-CoV-2 viral particles and/or anti-SARS-CoV-2 IgA-class immunoglobulins, wherein said biological sample is a saliva sample.
In particular, according to an aspect of the invention, the detection of SARS-CoV-2 viral particles and/or anti-SARS-CoV-2 IgA-class immunoglobulins in said biological sample corresponds to a positive diagnosis of SARS-CoV-2 infection.
Moreover, according to an aspect of the invention, the detection of anti-SARS-CoV-2 IgA-class immunoglobulins in said biological sample represents a positive index of the development of an immune response to SARS-CoV-2 virus.
The saliva sample subjected to analysis according to the method of the present invention is preferably a saliva sample of the posterior oropharynx, or a sample of oropharynx secretions. According to an aspect of the invention, said saliva sample can be obtained from an asymptomatic subject, or from a subject who already has the symptoms of SARS-CoV-2 infection, for example symptoms of moderate entity, such as fever, cough, shortage of breath, gastrointestinal disorders or more serious symptoms, such as pneumonia.
As already mentioned, the external layer of SARS-CoV-2 viral particles mainly consists of glycoproteins Spike, constituted by two subunits, S1 and S2, organized to form trimeric structures on the virion surface.
Therefore, according to an aspect of the present invention, the detection of said SARS-CoV-2 viral particles in said saliva sample is based upon the detection of one or more antigens of SARS-CoV-2 virus, in particular upon the detection of S1 subunits of SARS-CoV-2 Spike proteins and/or S2 subunits of SARS-CoV-2 Spike proteins.

The detection of said one or more SARS-CoV-2 antigens, and/or of anti-SARS-CoV-2 IgA-class immunoglobulins, can be performed by using reagents which are capable of binding selectively said one or more antigens and/or said IgA-class immunoglobulins by forming a complex, and by using means capable of detecting the formation of said complex, for example by generating a signal which can be easily detected to the naked eye.
According to an aspect of the invention, said detection, in a saliva sample, of one or more antigens of SARS-CoV-2 virus, can be performed by agglutination tests. Agglutination is a macroscopic phenomenon which can be seen to the naked eye on a light or transparent background and does not request any technical instruments. A first requirement necessary so that agglutination can take place is that the antigen to be detected is present in several copies repeated on the micro-organism, requirement met by SARS-CoV-2 Spike proteins. A second requirement is that the virus is present in high content in the biological sample to be analysed. In a preferred aspect of the invention, said agglutination test is a latex beads agglutination test (Latex Agglutination Test). In this specific test, the presence of antigen is highlighted by agglutination ("clumping") of (generally blue or black) latex particles coated with specific antibodies against the antigen itself.
According to an aspect of the invention, said latex beads can be coated with anti-SARS-CoV-2 antibodies. Preferably, said latex beads can be coated with anti-SARS-CoV-2 antibodies in concentration comprised between 2 and 0.2 mg per mg of latex.
According to a preferred aspect of the invention, said anti-SARS-CoV-2 antibodies are anti-SARS-CoV-2 Spike protein antibodies. In particular, said anti-SARS-CoV-2 antibodies can be selected among anti-SARS-CoV-2 Spike protein S1-subunit antibodies, and/or anti-SARS-CoV-2 Spike protein S2-subunit antibodies.
Not limiting examples of commercially available anti-SARS-CoV-2 antibodies which can be used in the herein described methods and kits include anti-SARS-CoV-2 Spike protein polyclonal rubber antibodies (#434243 MyBioSource, or Cat. Nr.: 3525 ProSci Inc.), anti-SARS-CoV-2 Spike protein monoclonal rubber antibodies (40150-R007 Sino Biological), or anti-SARS-CoV-2 Spike protein monoclonal murine antibodies (#GTX632604 GenTex).
The preparation of the latex beads coated with antibodies, or "sensitized", according to one of the embodiments of the present invention, can be performed by using any one of the standard procedures known in the art, for example by using commercially available kits and/or antibody reagents, according to the indications of the respective providers. In particular, the addition of bovine serum albumin (BSA) to the suspension of latex beads can be useful in increasing the stability of the conjugated and in blocking the non-specific bond sites on the bead surface.
According to an aspect of the invention, said latex bead agglutination method, comprises the following steps: i) putting in contact said saliva sample for a period of time of at least 5 minutes, preferably comprised between 2 and 5 minutes with said latex beads, ii) detecting the appearance of agglutination, therefore the appearance of agglutination indicates the presence of one or more antigens of SARS-CoV-2 virus, and by extension of potentially infecting virions, in said saliva sample to be analysed.
In order to check that the observed agglutination process really indicates the presence of one or more antigens of SARS-CoV-2 virus inside the saliva sample to be analysed, and on the contrary is not bound to a non-specific aggregation process, or "false positive", the method of the present invention can provide a step wherein the agglutination, by means of latex beads, of a control sample is evaluated.
Therefore, an aspect of the present invention relates to a method according to any one of the previously described embodiments, wherein a first volume of reagent comprising said latex beads is put in contact with a volume of said saliva sample to be analysed, and a second volume of reagent comprising said latex beads is put in contact with a volume of a control sample for a period of time of at least 5 minutes, preferably comprised between 2 and 5 minutes, and wherein the appearance of agglutination in said biological sample to be analysed and the absence of agglutination in said control sample indicate the presence of one or more antigens of SARS-CoV-2 virus in said biological sample to be analysed.
A suitable control sample according to the present invention, for example, is a suspension wherein said antigens of SARS-CoV-2 virus are absent. In order to easily display agglutination, the method of the present invention according to any one of herein described embodiments can include the use of one or more slides and/or one or more cardboards for agglutination tests.

Slides which can be used for the agglutination test according to the present invention are for example, slides characterized by two concavities, so that the agglutination test on the sample to be analysed and on the (surely negative) control sample can be performed simultaneously side by side.
In an embodiment, for example, a volume equal to 15 microlitres of suspension of sensitized latex beads can be placed on the concave surface of a suitable microscopy slide, near an equal volume of the saliva sample to be tested. The two drops of liquid can be mixed at first with the end of a sterile tip and then with a light orbital rotation motion of the whole slide. The possible agglutination reaction will appear as an irregular distribution of the beads in aggregates (clumping). In order to ease the display of the aggregates it is also possible to observe the slide against the background of a light cardboard. A preferred embodiment of the method is illustrated by way of example in Figure 1.
According to an additional aspect of the present invention, said detection, in a saliva sample, of one or more antigens of SARS-CoV-2 virus, can be performed by means of lateral flow immunochromatography test (Lateral Flow Assay, LFA).
In particular, according to an aspect of the invention, said lateral flow immunochromatography test comprises the steps of:
- i) contacting a volume of a solution comprising said saliva sample to be analysed with a solid support; said solid support is characterized by (a) a first "capture" area comprising an immobilized capture agent, capable of forming a complex with one or more SARS-CoV-2 antigens and by (b) a second "control" area comprising an immobilized control agent capable of forming a complex with a detection agent;
- ii) diagnosing SARS-CoV-2 infections when a signal visible to the naked eye appears in the first "capture" area and a signal visible to the naked eye appears in the second "control" area. Under visible to the naked eye for example coloured is meant so as to be detected to the sight.

Solid supports suitable to be used in the lateral flow immunochromatography test of the present invention are for example supports comprising a membrane and/or paper through which the solution of the sample to be analysed can migrate by capillary action. Not limiting examples of said solid supports include for example membrane made of nitrocellulose, Cellulose Acetate, polyvinylidene fluoride (PVDF), Nylon and polyethersulfone (PES), different from each other by porosity and electric charge. Said supports can have a shape suitable to carry out the test, for example, a strip-like shape. The detection agent used in the lateral flow immunochromatography test according to the present invention can be added to the solution comprising said saliva sample to be analysed or, alternatively, can be directly incorporated on/in said solid support.
In particular, according to an aspect of the invention, said detection agent can be incorporated on/in said solid support on/in an area preceding said first "capture" area. In this way, the present analyte of the solution of the biological sample to be analysed, by migrating through said solid support, meets and interacts with said detection agent, to be then subsequently blocked, and then detected, on/in said "capture" area.
Said detection agent can be in liquid form or in dried form, preferably it is in dried form if incorporated on/in said solid support.
A detection agent suitable to be used in the lateral flow immunochromatography test for the detection of one or more SARS-CoV-2 antigens according to one of the embodiments of the present invention, is a compound capable of binding one or more antigens of SARS-CoV-2 virus, conjugated to a marker agent.
In a preferred aspect of the invention, said detection agent is an anti-SARS-CoV-2 antibody, conjugated to a marker agent.
Not limiting examples of marker agents which can be conjugated to said detection agent, include latex beads, or colloidal gold nanoparticles with a diameter between 10 and 80 nm.
In a preferred embodiment of the invention, said marker agent is a gold nanoparticle with diameter equal to 40 nm.

The first "capture" area and the second "control" area as described in the present invention, can be implemented on the solid support selected for lateral flow immunochromatography test, in a shape selected between circular spot or line.
An immobilized capture agent suitable for the formation of a complex with one or more SARS-CoV-2 antigens, can be, for example, an anti-SARS-CoV-2 antibody. Preferably, said anti-SARS-CoV-2 antibody is an anti-SARS-CoV-2 Spike protein antibody.
An immobilized control agent suitable for the formation of a complex with one of the indicated detection agents can be an antibody capable of recognizing the Fc portion (common, not binding the antigen) of the detection antibody (ex. an anti-rat Fc antibody if the detector agent is an antibody generated in rat).
In a preferred embodiment of the lateral flow immunochromatography test for the detection of one or more SARS-CoV-2 antigens, said immobilized capture agent is an anti-SARS-CoV-2 Spike protein S1-subunit antibody; said immobilized control agent is an anti-rat IgG polyclonal antibody (portion Fc); and said detection agent is an anti-SARS-CoV-2 Spike protein S1-subunit rat monoclonal antibody, conjugated to a gold nanoparticle having diameter of 40 nm.
The solid supports for lateral flow immunochromatography test according to one of the embodiments described in the present invention, can be manufactured by using one or more commercially available kits and/or by using industrial techniques known in the art.

As already mentioned, one of the advantages of the present invention consists in the possibility of combining the detection of one or more antigens of SARS-CoV-2 virus in a saliva sample, to the detection of anti-SARS-CoV-2 IgA-class immunoglobulins in the same sample, therefore by providing a reliable and robust diagnostic method for identifying SARS-CoV-2 infection and detecting a specific mucosal immunity.
According to an aspect of the present invention, said detection of anti-SARS-CoV-2 IgA-class immunoglobulins can be performed by using tests analogous to those used for the detection of one or more previously described SARS-CoV-2 antigens, such as, for example, agglutination test and/or lateral flow immunochromatography test.

Said detection of anti-SARS-CoV-2 IgA-class immunoglobulins, for example, can be performed by means of a latex beads agglutination test. In this case, said latex beads can be coated, or sensitized, with native or recombinant SARS-CoV-2 virus antigens.
According to an aspect of the invention, said latex beads are coated with native or recombinant SARS-CoV-2 virus antigens in concentration comprised between 0.2 and 2 mg per mg of latex. In particular, a preferred aspect of the invention relates to an agglutination test by means of latex beads for the detection of anti-SARS-CoV-2 IgA-class immunoglobulins, wherein said beads are coated with S1 subunits of SARS-CoV-2 Spike proteins, said S1 subunits are fused with the Fc portion of the mouse IgG1 immunoglobulin. A not limiting example of SARS-CoV-2 Spike protein S1-subunits usable for coating said latex beads, is represented by recombinant Spike protein S1-subunits wherein the sequence 1-685 of S1-subunit is fused with the Fc portion of the mouse IgG1 immunoglobulin (Cat: 40591-V05H1, Sino Biological).
According to an additional aspect of the invention, said agglutination test by means of latex beads for the detection of anti-SARS-CoV-2 IgA-class immunoglobulins, comprises the following steps: i) contacting said saliva sample for a period of time of at least 2 minutes, preferably comprised between 2 and 5 minutes with said latex beads, ii) detecting the appearance of agglutination, therefore the appearance of agglutination indicates the presence of anti-SARS-CoV-2 IgA-class immunoglobulins in said saliva sample to be analysed.
In order to confirm the correct operation of the agglutination procedure for the detection of anti-SARS-CoV-2 IgA-class immunoglobulins, even in this case it is possible to perform the test on a "control" sample. A suitable "control" sample for example is a suspension wherein said anti-SARS-CoV-2 IgA-class immunoglobulins are absent. Alternatively, as control it is possible to use beads without SP1 antigen (empty beads).
Therefore, an aspect of the invention relates to a method for detecting anti-SARS-CoV-2 IgA-class immunoglobulins by means of latex beads agglutination test, wherein a first volume of reagent comprising said latex beads is put in contact with a volume of said biological sample to be analysed, and a second volume of reagent comprising said latex beads is put in contact with a volume of a control sample for a period of time of at least 2 minutes, preferably comprised between 2 and 5 minutes, and wherein the appearance of agglutination in said saliva sample to be analysed and the absence of agglutination in said control sample indicate the presence of anti-SARS-CoV-2 IgA-class immunoglobulins in said saliva sample to be analysed.
Although the secretory IgA are the most represented antibodies in the saliva (5-15 mg/dl, against 1.5 mg/dl of IgG and 0.5 of IgM), and the dimeric structure increases the agglutinating capability thereof (4 sites of bound for the antigen on each dimer), it is possible that the amount of anti SP1 specific antibodies existing in the oropharynx fluid results to be however insufficient to determine a macroscopically appreciable reaction. Therefore, according to an aspect of the invention, with the purpose of increasing the agglutinating power of the secretory IgA existing in the sample, said reagent volume comprising the latex beads, can include even an agglutinating agent, such as IgA-class human anti-immunoglobulin antibodies, for example a direct antibody against the Fc portion of human IgA.
The agglutination test by means of latex beads for the detection of anti-SARS-CoV-2 IgA-class immunoglobulins in one of the previously described embodiments, comprises even in this case one or more slides and/or one or more cardboards for agglutination test. A preferred embodiment of the method is illustrated by way of example in Figure 3. As mentioned, the detection of anti-SARS-CoV-2 IgA-class immunoglobulins can be performed even by means of lateral flow immunochromatography test.
According to an aspect of the invention, said lateral flow immunochromatography test for the detection of anti-SARS-CoV-2 IgA-class immunoglobulins, comprises the steps of:
- i) contacting a volume of a solution comprising said saliva sample to be analysed with a solid support; said solid support is characterized by (a) a first "capture" area comprising an immobilized capture agent, capable of forming a complex with said anti-SARS-CoV-2 IgA-class immunoglobulins, and by (b) a second "control" area comprising an immobilized control agent capable of forming a complex with a detection agent;
- ii) diagnosing the presence of anti-SARS-CoV-2 salivary antibodies when a signal visible to the naked eye appears in the first "capture" area and a signal visible to the naked eye appears in the second "control" area.
Solid supports suitable to be used in the just described immunochromatography test can be selected among the examples already mentioned in the present description.
Analogously, the detection agent used in said lateral flow immunochromatography test for the detection of anti-SARS-CoV-2 IgA-class immunoglobulins, can be added to the solution comprising said saliva sample to be analysed or, alternatively, can be directly incorporated on/in said solid support, according to one of the already described modes. A detection agent suitable to be used in said immunochromatography test for the detection of anti-SARS-CoV-2 IgA-class immunoglobulins, according to an aspect of the invention, is an agent capable of binding said anti-SARS-CoV-2 IgA-class immunoglobulins.
In particular, in a preferred aspect of the invention, said detection agent is the S1 subunit of SARS-CoV-2 Spike proteins, fused with the Fc portion of the mouse IgG1 immunoglobulin, wherein said S1 subunit is conjugated to a marker agent. A not limiting example of S1 subunit of SARS-CoV-2 Spike proteins usable as detection agent is represented by S1 subunit of recombinant Spike proteins wherein the sequence 1-685 of the S1 subunit is fused with the Fc portion of the mouse IgG1 immunoglobulin (Cat: 40591-V05H1, Sino Biological); said subunit is conjugated to a marker agent.
Said marker agent can be again selected among latex beads, or gold nanoparticles with a diameter between 10 and 80 nm, preferably it is a gold nanoparticle with diameter equal to 40 nm.
An immobilized capture agent suitable for the detection of IgA-class immunoglobulins by means of lateral flow immunochromatography test, is an anti-lgA-class immunoglobulin antibody. In particular, in an aspect of the invention, said immobilized capture agent is an anti-lgA-class monoclonal or polyclonal immunoglobulin antibody, preferably it is an anti-portion Fc antibody of human IgA immunoglobulin.
Not limiting examples of commercially available anti-lgA-class immunoglobulin antibodies which can be used as capture agents according to the method of the present invention, comprise [1H9] anti-lgA human monoclonal murine antibodies (ab7383, Abcam), anti- human IgA murine antibodies, clone 15D6, capable of recognizing an epitope contained in the domain CH2 of the fragment Fc of human IgA (# 5104-3104 Bio-Rad), or anti-human secretory IgA polyclonal goat antibodies (20-IG02, Fitzgerald).
In particular, in an embodiment of the invention, said immobilized capture agent is an anti-lgA-class immunoglobulin antibody; said immobilized control agent is an anti-portion Fc antibody of the mouse IgG1 immunoglobulin; and said detection agent is the S1 subunit of SARS-CoV-2 Spike proteins fused with the Fc portion of the mouse IgG1 immunoglobulin, wherein said S1 subunit is conjugated to a gold nanoparticle of 40 nm. In an alternative embodiment of the immunochromatography test for the detection of anti-SARS-CoV-2 IgA-class immunoglobulins, said solution comprising said saliva sample to be analysed includes anti-lgA-class immunoglobulins antibodies labelled with Ulfa-tag capable of forming a complex with said IgA-class immunoglobulins; said immobilized capture agent is an anti-Ulfa-tag antibody; said immobilized control agent is streptavidin; said detection agent comprises a first detection agent consisting of biotin-labelled gold nanoparticles capable of binding said immobilized control agent, and a second detection agent consisting of the S1 subunit of SARS-CoV-2 Spike proteins fused with the Fc portion of the mouse IgG1 immunoglobulin, conjugated to a 40-nm gold nanoparticle, said S1 subunit is capable of forming a complex with said IgA-class immunoglobulins. A first and a second preferred embodiments of the method are illustrated by way of example in Figure 4.
All tests according to any one of the embodiments of the present invention can be performed on the same saliva sample parallelly, or in sequential manner, to obtain a more accurate diagnosis.
As already mentioned, all tests for the detection of anti-SARS-CoV-2 IgA-class immunoglobulins according to any one of the embodiments of the present invention, can be used even for evaluating the development of immunity to SARS-CoV-2 virus, in particular for evaluating the development of mucosal immunity to SARS-CoV-2 virus.
In case the sensitivity limits of the method, in one of the herein described embodiments thereof, made necessary the concentration of the sample, integrating portion of the present invention, in any one of the embodiments thereof, it will be even a suitable concentrator device easy to be used at home.
Such device is based upon the principle of the direct osmosis (forward osmosis), and in particular upon the possibility of moving away most part of water and of solutes with low molecular weight contained in the salivary fluid (but not the micro-organisms or macromolecules such as the antibodies, which then result to be enriched) through the osmotic drawing by a concentrated solution (draw solution) of polyethylene oxide (PEO) with high molecular weight (>200 kDa) through a half-permeable membrane. To this purpose it will be possible to use a common cellulose dialysis membrane with "cut-off" of 10 kDa easily available on the market.
Therefore, the method of the present invention, in any one of the embodiments thereof, can include an additional concentration step of said biological sample preceding said detection step. Said concentration step can be performed by means of direct osmosis, preferably by means of a cellulose membrane with cut-off equal to 10 kDa.
The osmotic process allows to obtain a significative concentration of the salivary sample (equal to about 4-5 times compared to the initial concentration), within few minutes (from five to ten minutes).
Examples of devices suitable for the concentration of the salivary sample according to the present invention, include a "sachet" consisted of a dialysis pipe segment partially filled up with dry or highly concentrated PEO, to be dipped in the beaker containing the sample to be concentrated, or a container constituted by two chambers, one containing the PEO solution and the other one the sample, separated by the dialysis membrane arranged vertically or horizontally (Figure 2).

Object of the present invention is also a kit for diagnosing SARS-CoV-2 infection comprising one or more means for the detection, by agglutination tests, of SARS-CoV-2 viral particles in a saliva sample. Said kit can include agglutination means coated with a reagent capable of binding one or more antigens of SARS-CoV-2 virus, according to any one of the previously described embodiments.

An example of reagent capable of binding one or more antigens of SARS-CoV-2 virus is represented by anti-SARS-CoV-2 antibodies, in particular anti-SARS-CoV-2 Spike protein antibodies. In a preferred embodiment, said anti-SARS-CoV-2 Spike protein antibodies are selected among anti-SARS-CoV-2 Spike protein S1-subunit antibodies or anti-SARS-CoV-2 Spike protein S2-subunit antibodies.
In an embodiment of the kit, said agglutination means are latex beads. Said kit can also include one or more solid supports for agglutination tests according to one of the previously described embodiments.

An additional aspect of the present invention relates to a kit for diagnosing SARS-CoV-2 infection comprising one or more means for the detection, by means of lateral flow immunochromatography test (LFA), of SARS-CoV-2 viral particles in a saliva sample. Said kit can include said solid support means selected among the previously described ones, capable of absorbing said saliva sample by capillary action. These solid support means can be characterized by (a) a first "capture" area comprising an immobilized capture agent, capable of forming a complex with one or more SARS-CoV-2 antigens and by (b) a second "control" area comprising an immobilized control agent capable of forming a complex with a detection agent.
Preferably, according to an embodiment, said solid support means are in form of strip. In particular, said first "capture" area and said second "control" area can be implemented in form of circular spot or line.
According to an aspect of the invention, said solid support means can be characterized by (c) a third area preceding said "capture" area, comprising a detection agent in dehydrated form.
According to an additional aspect of the invention, said detection agent can even be incorporated in the kit under the form of one or more pre-packaged aliquots.
A detection agent suitable to be used in the present test is a detection agent capable of binding one or more antigens of SARS-CoV-2 virus conjugated to a marker agent, according to any one of the previously described embodiments.

In a preferred embodiment, said detection agent is an anti-SARS-CoV-2 antibody conjugated to a marker agent, wherein said marker agent is a gold nanoparticle with a diameter between 10 e 80 nm.
According to an aspect of the invention, said immobilized capture agent is an anti-SARS-CoV-2 antibody, preferably is an anti-SARS-CoV-2 Spike protein antibody.
According to an aspect of the invention, said immobilized "control" agent is a polyclonal antibody capable of recognizing directly the detection agent.
In particular, in a preferred embodiment of the kit, said immobilized capture agent is an anti-SARS-CoV-2 Spike protein S1-subunit antibody; said immobilized control agent is an anti-rat IgG antibody, portion Fc; and said detection agent is an anti-SARS-CoV-2 Spike protein S1-subunit rat monoclonal antibody conjugated to a gold nanoparticle having diameter of 40 nm.

Object of the present invention is also a kit for diagnosing SARS-CoV-2 infection comprising one or more means for the detection, by agglutination test of anti-SARS-CoV-2 IgA-class immunoglobulins in a saliva sample.
In this case, said agglutination means are coated with a reagent capable of binding said anti-SARS-CoV-2 IgA-class immunoglobulins in a saliva sample, according to one of the previously described embodiments.
In a preferred embodiment of the invention, said reagents capable of binding said anti-SARS-CoV-2 IgA-class immunoglobulins are S1 subunits of SARS-CoV-2 Spike proteins, fused with the Fc portion of the mouse IgG1 immunoglobulin.
In an embodiment of the kit, said agglutination means are latex beads. Said kit can also comprise one or more solid supports for agglutination tests according to one of the previously described embodiments.
Said kit can also be used for evaluating the immune system response to SARS-CoV-2 virus.

Object of the present invention is also a kit for diagnosing SARS-CoV-2 infection comprising one or more means for the detection by means of lateral flow immunochromatography test (LFA) of anti-SARS-CoV-2 IgA-class immunoglobulins in a saliva sample. Said kit can include said solid support means selected among the previously described ones, capable of absorbing said saliva sample by capillary action.
These solid support means can be characterized by (a) a first "capture" area comprising an immobilized capture agent, capable of forming a complex with anti-SARS-CoV-2 IgA-class immunoglobulins and by (b) a second "control" area comprising an immobilized control agent capable of forming a complex with a detection agent. Preferably, according to an embodiment of the invention, said solid support means are in form of strip. In particular, said first "capture" area and said second "control" area can be implemented in form of circular spot or line.
According to an aspect of the invention, said solid support means can also be characterized by (c) a third area preceding said "capture" area, wherein said third area comprises a dehydrated detection agent.
According to an additional aspect of the invention, said detection agent can even be incorporated in the kit under the form of one or more pre-packaged aliquots.
A detection agent suitable to be used in the present test is an agent capable of binding anti-SARS-CoV-2 IgA-class immunoglobulins, conjugated to a marker agent, according to any one of the previously described embodiments.
In a preferred embodiment, said detection agent is the S1 subunit of SARS-CoV-2 Spike proteins, fused with the Fc portion of the mouse IgG1 immunoglobulin, wherein said S1 subunit is conjugated to a marker agent.
Preferably, said marker agent is a gold nanoparticle with a diameter between 10 and 80 nm.
According to an aspect of the invention, said immobilized capture agent is an anti-lgA-class immunoglobulin antibody, preferably it is an anti-lgA-class immunoglobulin monoclonal or polyclonal, generated in mouse or goat.
According to an aspect of the invention, said immobilized "control" agent is an anti-portion Fc antibody of the mouse IgG1 immunoglobulin.
In a preferred embodiment of the kit, said immobilized capture agent is an anti-lgA-class immunoglobulin antibody; said immobilized control agent is an anti-portion Fc antibody of the mouse IgG1 immunoglobulin; and said detection agent is the S1 subunit of SARS-CoV-2 Spike proteins fused with the Fc portion of the mouse IgG1 immunoglobulin, wherein said S1 subunit is conjugated to a gold nanoparticle of 40 nm.
Said kit can further include one or more aliquots comprising anti-lgA-class immunoglobulin antibodies labelled with Ulfa-tag capable of forming a complex with said IgA-class immunoglobulins.
In this case, in another embodiment of the kit (Figure 4a) said immobilized capture agent is an anti-Ulfa-tag antibody; said immobilized control agent is streptavidin; said detection agent comprises a first detection agent consisting of biotin-labelled gold nanoparticles capable of binding said immobilized control agent, and a second detection agent consisting of the S1 subunit of SARS-CoV-2 Spike proteins fused with the Fc portion of the mouse IgG1 immunoglobulin, conjugated to a 40-nm gold nanoparticle, said S1 subunit is capable of forming a complex with said IgA-class immunoglobulins.
Said kit in any one of the embodiments thereof can even be used for the evaluation of the immune response to SARS-CoV-2 virus.

An additional aspect of the present invention relates to the combination of at least a kit suitable for the detection of SARS-CoV-2 viral particles in a saliva sample, and at least a kit suitable for the detection of anti-SARS-CoV-2 immunoglobulins in said saliva sample, according to any one of the previously described embodiments, for a more accurate diagnosis of SARS-CoV-2 infections, and/or for evaluating the immune system response to SARS-CoV-2 virus.
The kits according to any one of the embodiments of the present invention can further include a device for concentrating said saliva sample according to any one of the previously described embodiments, wherein said device include a dialysis membrane with a cut-off equal to 10 kDa.

Some embodiments of the present invention are reported hereinafter:
1. A method for diagnosing SARS-CoV-2 infection comprising a step of detecting in a biological sample SARS-CoV-2 viral particles and/or anti-SARS-CoV-2 IgA-class immunoglobulins, wherein said biological sample is a saliva sample.
2. The method according to embodiment 1, wherein said biological sample is obtained from a subject with symptoms of SARS-CoV-2 infection or from an asymptomatic subject.
3. The method according to embodiments 1 or 2, wherein the detection of said SARS-CoV-2 viral particles consists in the detection of one or more antigens of SARS-CoV-2 virus, wherein said one or more antigens are S1 subunits of SARS-CoV-2 Spike proteins and/or S2 subunits of SARS-CoV-2 Spike proteins.
4. The method according to any one of embodiments 1 to 3, wherein said step of detecting SARS-CoV-2 viral particles is carried out by means of agglutination test.
5. The method according to embodiment 4, wherein said agglutination test is a latex beads agglutination test (Latex Agglutination Test, LAT).
6. The method according to embodiment 5, wherein said latex beads are coated with anti-SARS-CoV-2 antibodies.
7. The method according to embodiment 6, wherein said latex beads are coated with anti-SARS-CoV-2 antibodies in concentration comprised between 0.2 and 2 mg per mg of latex.
8. The method according to embodiments 6 or 7, wherein said anti-SARS-CoV-2 antibodies are anti-SARS-CoV-2 Spike protein antibodies.
9. The method according to embodiment 8, wherein said anti-SARS-CoV-2 antibodies are selected among anti-SARS-CoV-2 Spike protein S1-subunit antibodies, and/or anti-SARS-CoV-2 Spike protein S2-subunit antibodies.
10. The method according to any one of embodiments 5 to 9 comprising the following steps: i) contacting said saliva sample for a period of time between 2 and 5 minutes with said latex beads, ii) detecting the appearance of agglutination, therefore the appearance of agglutination indicates the presence of SARS-CoV-2 viral particles in said saliva sample to be analysed.
11. The method according to any one of embodiments 5 to 10, wherein a first volume of reagent comprising said latex beads is put in contact with a volume of said biological sample to be analysed, and a second volume of reagent comprising said latex beads is put in contact with a volume of a control sample for a period of time between 2 and 5 minutes, and wherein the appearance of agglutination in said biological sample to be analysed and the absence of agglutination in said control sample indicate the presence of SARS-CoV-2 viral particles in said biological sample to be analysed.
12. The method according to embodiment 11, wherein said control sample comprises a suspension wherein said SARS-CoV-2 virus antigens are absent.
13. The method according to any one of embodiments 5 to 12, wherein in said latex beads agglutination test one or more slides and/or one or more cardboards for agglutination test are used.
14. The method according to any one of embodiments 1 to 3, wherein said step of detecting SARS-CoV-2 viral particles is carried out by means of lateral flow immunochromatography test (Lateral Flow Assay, LFA).
15. The method according to embodiment 14, wherein said lateral flow immunochromatography test comprises the steps of:
   - i) contacting a volume of a solution comprising said saliva sample to be analysed with a solid support; said solid support is characterized by (a) a first "capture" area comprising an immobilized capture agent, capable of forming a complex with one or more SARS-CoV-2 antigens and by (b) a second "control" area comprising an immobilized control agent capable of forming a complex with a detection agent;
   - ii) diagnosing SARS-CoV-2 infections when a signal visible to the naked eye appears in the first "capture" area and a signal visible to the naked eye appears in the second "control" area.
16. The method according to embodiment 15, wherein a solid support is used in said step i) comprising a membrane and/or paper through which the solution comprising said saliva sample to be analysed migrates by capillary action.
17. The method according to embodiments 15 or 16, wherein said solid support is selected among membrane made of nitrocellulose, Cellulose Acetate, polyvinylidene fluoride (PVDF), Nylon and polyethersulfone (PES), different from each other by porosity and electric charge.
18. The method according to any one of embodiments 15 to 17, wherein said detection agent is contained in said solution comprising said saliva sample to be analysed.
19. The method according to any one of embodiments 15 to 17, wherein said detection agent is incorporated on/in said solid support.
20. The method according to embodiment 19, wherein said detection agent is incorporated on/in said solid support on/in an area preceding said first "capture" area.
21. The method according to any one of embodiments 15 to 20, wherein said detection agent is in dried form.
22. The method according to any one of embodiments 15 to 21, wherein said detection agent is capable of binding one or more antigens of SARS-CoV-2 virus.
23. The method according to any one of embodiments 15 to 22, wherein said detection agent is an anti-SARS-CoV-2 antibody, said antibody is conjugated to a marker agent.
24. The method according to embodiment 23, wherein said marker agent is a gold nanoparticle with a diameter between 10 and 80 nm.
25. The method according to embodiments 23 or 24, wherein said marker agent is a colloidal gold nanoparticle having diameter equal to 40 nm.
26. The method according to any one of embodiments 15 to 25, wherein said first "capture" area and said second "control" area are in form of circular spot or line.
27. The method according to any one of embodiments 15 to 26, wherein said immobilized capture agent is an anti-SARS-CoV-2 antibody.
28. The method according to embodiment 27, wherein said anti-SARS-CoV-2 antibodies are anti-SARS-CoV-2 Spike protein antibodies.
29. The method according to any one of embodiments 15 to 28, wherein said immobilized control agent is an antibody capable of recognizing the Fc portion (common, not binding the antigen) of the detection antibody (ex. an anti-rat Fc antibody if the detection agent is an antibody generated in the mouse).
30. The method according to any one of embodiments 15 to 29, wherein said immobilized capture agent is an anti-SARS-CoV-2 Spike protein S1-subunit antibody; said immobilized control agent is an anti-IgG rat mono or polyclonal antibody (portion Fc); and said detection agent is an anti-SARS-CoV-2 Spike protein S1-subunit rat monoclonal antibody conjugated to a gold nanoparticle with diameter of 40 nm.
31. The method according to any one of embodiments 1 to 30, wherein said detection of anti-SARS-CoV-2 IgA-class immunoglobulins is carried out by means of agglutination test.
32. The method according to any one of embodiments 1 to 31, wherein said detection of anti-SARS-CoV-2 IgA-class immunoglobulins is performed by means of latex beads agglutination test (Latex Agglutination Test, LAT).
33. The method according to embodiment 32 wherein said latex beads are coated with native or recombinant SARS-CoV-2 virus antigens.
34. The method according to embodiments 32 or 33, wherein said latex beads are coated with native or recombinant SARS-CoV-2 virus antigens in concentration comprised between 0.2 and 2 mg per mg of latex.
35. The method according to embodiments 33 or 34, wherein said latex beads are coated with S1 subunits of SARS-CoV-2 Spike proteins, said S1 subunits are fused with the Fc portion of the mouse IgG1 immunoglobulin.
36. The method according to any one of embodiments 32 to 35, comprising the following steps: i) contacting said saliva sample for a period of time between 2 and 5 minutes with said latex beads, ii) detecting the appearance of agglutination, therefore the appearance of agglutination indicates the presence of anti-SARS-CoV-2 IgA-class immunoglobulins in said saliva sample to be analysed.
37. The method according to any one of embodiments 32 to 35, wherein a first volume of reagent comprising said latex beads is put in contact with a volume of said biological sample to be analysed, and a second volume of reagent comprising said latex beads is put in contact with a volume of a control sample for a period of time between 2 and 5 minutes, and wherein the appearance of agglutination in said saliva sample to be analysed and the absence of agglutination in said control sample indicate the presence of anti-SARS-CoV-2 IgA-class immunoglobulins in said saliva sample to be analysed.
38. The method according to embodiment 37, wherein said control sample is a sample wherein said anti-SARS-CoV-2 IgA-class immunoglobulins are absent.
39. The method according to any one of embodiments 32 to 38, wherein said reagent volume comprising the latex beads, further includes anti-lgA-class human immunoglobulin antibodies.
40. The method according to any one of embodiments 32 to 39, wherein in said latex beads agglutination test one or more slides and/or one or more cardboards for agglutination test are used.
41. The method according to any one of embodiments 1 to 30, wherein said detection of anti-SARS-CoV-2 IgA-class immunoglobulins is performed by means of lateral flow immunochromatography test (Lateral Flow Assay, LFA).
42. The method according to embodiment 41, wherein said lateral flow immunochromatography test comprises the steps of:
   - i) contacting a volume of a solution comprising said saliva sample to be analysed with a solid support; said solid support is characterized by (a) a first "capture" area comprising an immobilized capture agent, capable of forming a complex with said anti-SARS-CoV-2 IgA-class immunoglobulins, and by (b) a second "control" area comprising an immobilized control agent capable of forming a complex with a detection agent;
   - ii) diagnosing the presence of anti-SARS-CoV-2 salivary antibodies when a signal visible to the naked eye appears in the first "capture" area and a signal visible to the naked eye appears in the second "control" area.
43. The method according to embodiment 42, wherein in said step i) a solid support is used comprising a membrane and/or paper through which the solution comprising said saliva sample to be analysed migrates by capillary action.
44. The method according to embodiments 42 or 43, wherein said solid support is selected among membrane made of nitrocellulose, Cellulose Acetate, polyvinylidene fluoride (PVDF), Nylon and polyethersulfone (PES), different from each other by porosity and electric charge.
45. The method according to any one of embodiments 42 to 44, wherein said detection agent is contained in said solution comprising said biological sample to be analysed.
46. The method according to any one of embodiments 42 to 44, wherein said detection agent is incorporated on/in said solid support.
47. The method according to embodiment 46, wherein said detection agent is incorporated on/in said solid support on/in an area preceding said first "capture" area.
48. The method according to any one of embodiments 42 to 47, wherein said detection agent is in dried form.
49. The method according to any one of embodiments 42 to 48, wherein said detection agent is capable of binding said anti-SARS-CoV-2 IgA-class immunoglobulins.
50. The method according to any one of embodiments 42 to 49, wherein said detection agent is the S1 subunit of SARS-CoV-2 Spike proteins, fused with the Fc portion of the mouse IgG1 immunoglobulin, said S1 subunit is conjugated to a marker agent.
51. The method according to embodiment 50, wherein said marker agent is a gold nanoparticle with a diameter between 10 and 80 nm.
52. The method according to embodiments 50 or 51, wherein said marker agent is a colloidal gold nanoparticle with diameter equal to 40 nm.
53. The method according to any one of embodiments 42 to 52, wherein said first "capture" area and said second "control" area are in form of circular spot or line.
54. The method according to any one of embodiments 42 to 53, wherein said immobilized capture agent is an anti-lgA-class immunoglobulin antibody.
55. The method according to embodiment 54, wherein said immobilized capture agent is an anti-lgA-class immunoglobulins monoclonal or polyclonal murine antibody.
56. The method according to any one of embodiments 42 to 55, wherein said immobilized control agent is an anti-portion Fc antibody of the mouse IgG1 immunoglobulin.
57. The method according to any one of embodiments 42 to 56, wherein said immobilized capture agent is an anti-lgA-class immunoglobulin antibody; said immobilized control agent is an anti-portion Fc antibody of the mouse IgG1 immunoglobulin; and said detection agent is the S1 subunit of SARS-CoV-2 Spike proteins fused with the Fc portion of the mouse IgG1 immunoglobulin, wherein said S1 subunit is conjugated to a gold nanoparticle of 40 nm.
58. The method according to any one of embodiments 42 to 56, wherein said solution comprising said saliva sample to be analysed includes anti-lgA-class immunoglobulin antibodies labelled with Ulfa-tag capable of forming a complex with said IgA-class immunoglobulins; said immobilized capture agent is an anti-Ulfa-tag antibody; said immobilized control agent is streptavidin; said detection agent comprises a first detection agent consisting of biotin-labelled gold nanoparticles capable of binding said immobilized control agent, and a second detection agent consisting of the S1 subunit of SARS-CoV-2 Spike proteins fused with the Fc portion of the mouse IgG1 immunoglobulin, conjugated to a 40-nm gold nanoparticle, said S1 subunit is capable of forming a complex with said IgA-class immunoglobulins.
59. The method according to any one of embodiments 1 to 58, comprising a concentration step of said biological sample preceding said detection step.
60. The method according to embodiment 59, wherein said concentration step is carried out by means of direct osmosis.
61. The method according to embodiments 59 or 60, wherein said concentration step is carried out by means of a membrane made of cellulose with cut-off equal to 10 kDa.
62. The method according to any one of embodiments 1 to 61, therefore the detection of antigens of SARS-CoV-2 virus and/or of anti-SARS-CoV-2 IgA-class immunoglobulins in said biological sample corresponds to a positive diagnosis of SARS-CoV-2 infection.
   The herein described methods according to any embodiment could be used apart from the diagnosis even for the detection of mucosal immunity to SARS-CoV-2 virus.
63. A kit for diagnosing SARS-CoV-2 infection comprising one or more means for the detection, by agglutination tests, of SARS-CoV-2 viral particles in a saliva sample.
64. The kit according to embodiment 63, comprising agglutination means coated with a reagent capable of binding one or more antigens of SARS-CoV-2 virus.
65. The kit according to embodiment 64, wherein said reagent capable of binding one or more antigens of SARS-CoV-2 virus is an anti-SARS-CoV-2 antibody.
66. The kit according to any one of embodiments 64 to 65, wherein said reagent capable of binding one or more antigens of SARS-CoV-2 virus is an anti- SARS-CoV-2 Spike protein antibody.
67. The kit according to any one of embodiments 64 to 66, wherein said reagent capable of binding one or more antigens of SARS-CoV-2 virus is an anti-SARS-CoV-2 Spike protein S1-subunit antibody or an anti-SARS-CoV-2 Spike protein S2-subunit antibody.
68. The kit according to any one of embodiments 64 to 67, wherein said agglutination means are latex beads.
69. The kit according to any one of embodiments 63 to 68, further comprising one or more solid supports for agglutination tests.
70. The kit for diagnosing SARS-CoV-2 infection comprising one or more means for the detection, by means of lateral flow immunochromatography test (LFA), of SARS-CoV-2 viral particles in a saliva sample.
71. The kit according to embodiment 70, further comprising one or more aliquots of a detection agent.
72. The kit according to embodiments 70 or 71, comprising solid support means capable of absorbing said saliva sample by capillary action; said solid support means are characterized by (a) a first "capture" area comprising an immobilized capture agent, capable of forming a complex with one or more SARS-CoV-2 antigens and by (b) a second "control" area comprising an immobilized control agent capable of forming a complex with a detection agent.
73. The kit according to embodiment 72, wherein said solid support means are characterized by (c) a third area preceding said "capture" area, comprising an immobilized detection agent.
74. The kit according to any one of embodiments 71 to 73, wherein said detection agent is capable of binding one or more SARS-CoV-2 virus antigens.
75. The kit according to any one of embodiments 71 to 74, wherein said detection agent is an anti-SARS-CoV-2 antibody, said antibody is conjugated to a marker agent.
76. The kit according to embodiment 75, wherein said marker agent is a gold nanoparticle with a diameter between 10 and 80 nm.
77. The kit according to any one of embodiments 72 to 76, wherein said immobilized "capture" agent is an anti-SARS-CoV-2 antibody.
78. The kit according to embodiment 77, wherein said antibody anti-SARS-CoV-2 is an anti-SARS-CoV-2 Spike protein antibody.
79. The kit according to any one of embodiments 72 to 78, wherein said immobilized "control" agent is an antibody capable or recognizing the Fc portion (common, not binding the antigen) of the detection antibody (ex. an anti-rat Fc antibody if the detection agent is an antibody generated in the rat).
80. The kit according to any one of embodiments 72 to 79, wherein said immobilized capture agent is an anti-SARS-CoV-2 Spike protein S1-subunit antibody; said immobilized control agent is an anti-rat IgG (portion Fc) antibody; and said detection agent is an anti-SARS-CoV-2 Spike protein S1-subunit rat monoclonal antibody conjugated to a gold nanoparticle having diameter of 40 nm.
81. The kit according to any one of embodiments 72 to 80, wherein said first "capture" area and said second "control" area are in form of circular spot or line.
82. The kit for diagnosing SARS-CoV-2 infection comprising one or more means for the detection, by agglutination tests, of anti-SARS-CoV-2 IgA-class immunoglobulins in a saliva sample.
83. The kit according to embodiment 82, comprising agglutination means coated with a reagent capable of binding anti-SARS-CoV-2 IgA-class immunoglobulins in a saliva sample.
84. The kit according to embodiments 82 or 83, wherein said reagent capable of binding anti-SARS-CoV-2 IgA-class immunoglobulins is constituted by native or recombinant SARS-CoV-2 virus antigens.
85. The kit according to any one of embodiments 82 to 84, wherein said reagent capable of binding said anti-SARS-CoV-2 IgA-class immunoglobulins is the S1 subunit of SARS-CoV-2 Spike proteins, fused with the Fc portion of the mouse IgG1 immunoglobulin.
86. The kit according to any one of embodiments 83 to 85, wherein said agglutination means are latex beads.
87. The kit according to any one of embodiments 83 to 86, comprising one or more solid supports for agglutination tests.
88. The kit for diagnosing SARS-CoV-2 infection comprising one or more means for the detection, by means of lateral flow immunochromatography test (LFA) of anti-SARS-CoV-2 IgA-class immunoglobulins in a saliva sample.
89. The kit according to embodiment 88, further comprising one or more aliquots of a detection agent.
90. The kit according to embodiments 88 or 89, comprising solid support means capable of absorbing said saliva sample by capillary action; said solid support means are characterized by (a) a first "capture" area comprising an immobilized capture agent, capable of forming a complex with anti-SARS-CoV-2 IgA-class immunoglobulins and by (b) a second "control" area comprising an immobilized control agent capable of forming a complex with a detection agent.
91. The kit according to embodiment 90, wherein said solid support means are characterized by (c) a third area preceding said "capture" area, comprising an immobilized detection agent.
92. The kit according to any one of embodiments 89 to 91, wherein said detection agent is capable of binding said anti-SARS-CoV-2 IgA-class immunoglobulins.
93. The kit according to any one of embodiments 89 to 92, wherein said detection agent is the S1 subunit of SARS-CoV-2 Spike proteins, fused with the Fc portion of the mouse IgG1 immunoglobulin, said S1 subunit is conjugated to a marker agent.
94. The kit according to embodiment 93, wherein said marker agent is a gold nanoparticle with a diameter between 10 and 80 nm.
95. The kit according to any one of embodiments 89 to 94, wherein said immobilized "capture" agent is an anti-lgA-class immunoglobulin antibody.
96. The kit according to embodiment 95, wherein said immobilized capture agent is an anti-lgA-class immunoglobulin monoclonal or polyclonal antibody.
97. The kit according to any one of embodiments 89 to 96, wherein said immobilized "control" agent is an anti-portion Fc antibody of the mouse IgG1 immunoglobulin.
98. The kit according to any one of embodiments 89 to 97, wherein said immobilized capture agent is an anti-lgA-class immunoglobulin antibody; said immobilized control agent is an anti-portion Fc antibody of the mouse IgG1 immunoglobulin; and said detection agent is the S1 subunit of SARS-CoV-2 Spike proteins fused with the Fc portion of the mouse IgG1 immunoglobulin, wherein said S1 subunit is conjugated to a gold nanoparticle of 40 nm.
99. The kit according to any one of embodiments 89 to 97, further comprising one or more aliquots of anti-lgA-class immunoglobulin antibodies labelled with Ulfa-tag capable of forming a complex with said IgA-class immunoglobulins.
100. The kit according to embodiment 99, wherein said immobilized capture agent is an anti-Ulfa-tag antibody; said immobilized control agent is streptavidin; said detection agent comprises a first detection agent consisting of biotin-labelled gold nanoparticles capable of binding said immobilized control agent, and a second detection agent consisting of the S1 subunit of SARS-CoV-2 Spike proteins fused with the Fc portion of the mouse IgG1 immunoglobulin, conjugated to a 40-nm gold nanoparticle, said S1 subunit is capable of forming a complex with said IgA-class immunoglobulins.
101. The kit according to any one of embodiments 89 to 100, wherein said first "capture" area and said second "control" area are in form of circular spot or line.
102. A kit combination comprising at least a kit according to any one of embodiments 63 to 81 and at least a kit according to any one of embodiments 82 a 101 for diagnosing SARS-CoV-2 infection and for evaluating the immune system response to SARS-CoV-2 virus.
103. The kit according to any one of embodiments 63 to 102, further comprising a device for concentrating said saliva sample.
104. The kit according to the preceding embodiment wherein said device includes a dialysis membrane with a cut-off equal to 10 kDa.

### EXAMPLES

Some not limiting examples of the methods according to the present invention are reported hereinafter.

### 1. Latex beads agglutination test for the detection of SARS-CoV-2 (Spike proteins) in the saliva

1.1. **Preparation of the antibody reagents:** the anti-SARS-CoV-2 Spike Protein, subunit 1 (SP1) (for options see page 9 line 19) antibodies are purified from possible preservatives and diluted at the concentration of 0.5 mg/ml in two different buffers (Reaction buffer A and B), by using the kit "Antibody Concentration and Clean Up Kit for Latex & Europium: Abcam" cat # ab269965. To this purpose 50 micrograms of each reagent are diluted inside the cartridge/filter in the wished buffer until a final volume of 500 microlitres and centrifuged at 15000g for 2-5 minutes at 4°C until obtaining a final volume of about 100 microlitres; after having brought the volume to 500 microlitres with the same buffer (A or B), this same procedure is repeated for further four times. At the end of the fifth washing the antibody concentration is determined by means of colorimetric method (Bradford) and brought (by means of additional centrifugation or dilution with the same buffer) to the final value of 0.5 mg/ml.
1.2. **Conjugation of the antibody to the latex beads**: Such procedure is carried out according to the instructions of kit Latex Conjugation kit -400 nm Black Abcam cat # ab26989, with small modifications. In order to detect, for each antibody, the optimum conditions of concentration and pH for the covalent bond to the beads, 6 reactions are prepared: 3 different concentrations (0.5, 0.1 and 0.05 mg/ml), each one in the two different buffers A and B at different pH. For each reaction, 40 microlitres of antibody are used to re-suspend accurately the lyophilized latex spheres at the bottom of the test tube provided by the kit; the antibody binding reaction to the beads is performed at room temperature for 15 minutes, after which each reaction will be interrupted with the addition of 1 ml of Quencer 1x, provided by the same kit. After 5 minutes at room temperature the test tubes will be centrifuged at 10000 rpm for 1 minute in a counter centrifuge, the supernatant removed and the beads further re-suspended in 40 microlitres of the storage buffer (storage buffer) existing in the kit. Alternatively, PBS could be used. In both cases bovine albumin (BSA) at the final concentration of 1 mg/ml (0.1%) is added. The latter increases the conjugate stability and further acts as blocking agent of the bond non-specific sites. The so-obtained suspension (1 % w/w of latex in PBS/BSA 0.1%) is kept at 4°C and used for the subsequent procedures.
1.3. **Agglutination reaction:** For the agglutination reaction, 15 microlitres of each one of the conjugates are deposited with a micropipette on the concave surface of a suitable microscopy slide, together with an equal volume of the sample to be tested. The two drops of liquid are mixed at first with the end of a sterile point and then with a light orbital rotation motion of the whole slide. The possible agglutination reaction appears like an irregular distribution of the beads in aggregates (clumping). Otherwise, the suspension keeps a homogenously lactescent aspect. The maximum reaction time is 5 minutes. In order to ease the display of the aggregates it is suitable to observe the slide against the background of a light cardboard. Slides including two concavities are used (Bioclass s.r.l., article code 01.4290.07), so that the "real" reaction (sample to be analysed) and the control one (surely negative sample) are performed simultaneously side by side.
1.4. **Negative control:** the negative control is represented by the simple culture medium, in which the viral particles are produced, or by a suspension of recombinant virus (MLV) not expressing the Spike protein, respectively.

### 2. Latex beads agglutination test for the detection of anti-SARS-Cov-2 S-glycoprotein specific secretory IgA (slgA) in the saliva

2.1. **Angigen preparation**: SARS-CoV-2 (2019-nCoV) Spike S1-mFc Recombiant Protein. Cat: 40591-V05H1, (Sino Biological) recombinant antigen, at first in lyophilized form by PBS sterile pH 7.4 containing Trehalose and Mannitol as stabilizers, is purified by the preservative agents and diluted at the concentration of 0.5 mg/ml in two different buffers (Reaction buffer A and B) by using the "Antibody Concentration and Clean Up Kit for Latex & Europium: Abcam cat # ab269965" kit. To this purpose 50 micrograms of antigen (reconstituted in dH₂O) are diluted inside the cartridge/filter in the wished buffer until a final volume of 500 microlitres and centrifuged at 15000g for 2-5 minutes at 4°C, until obtaining a final volume of about 100 microlitres; after having brought the volume back to 500 microlitres with the same buffer (A or B), this same procedure is repeated for other four times. At the end of the fifth washing the antibody concentration is determined by means of colorimetric method (Bradford) and adjusted (by means of further centrifugation or dilution with the same buffer) to the final value of 0.5 mg/ml.
2.2. **Conjugation of antigen to the latex beads:** Such procedure is performed according to the instruction of the "Latex Conjugation kit-400 nm Black (Abcam cat # ab26989)" kit, with small modifications. In particular, 6 reactions are prepared: 3 different concentrations (0.5, 0.1 and 0.05 mg/ml) of protein, each one in the two different buffers A and B. For each reaction, 40 microlitres of each antigen solution (equal to 20, 4 and 2 micrograms, respectively) are used to re-suspend accurately the lyophilized latex beads at the bottom of the test tube provided by the kit; the (covalent) bond reaction of antigen to the beads is performed at RT for 15 minutes, after which each reaction is interrupted with the addition of 1 ml of Quencer 1x, provided by the same kit. After 5 minutes at room temperature the test tubes are centrifuged at 10000 rpm for 1 minute in a counter centrifuge, the supernatant removed and the beads further re-suspended in 40 microlitres of the storage buffer (storage buffer) existing in the kit. Alternatively, simple PBS can be used. In both cases bovine albumin (BSA) at the final concentration of 1 mg/ml (0.1%) is added. The latter increases the conjugate stability and also acts as agent blocking the bond non-specific sites. The so-obtained suspension (1 % w/w of latex in PBS/BSA 0.1 %) is kept at 4°C and used for the subsequent procedures.
2.3. **Agglutination reaction:** For the agglutination reaction the protocol described at point 1.3 is followed.

### 3. Lateral flow immunochromatography test (LFA) for the detection of anti SARS-Cov-2 S-glycoprotein specific secretory IgA (slgA) in the saliva

According to a first embodiment, the test can be implemented by using a commercial kit "Universal Lateral Flow Assay Kit, Expedeon 4300-0100".
3.1. **Preparation of the capture agent (anti human IgA conjugated with Ulfa-tag)** The anti-human IgA antibody (for options see page 17 line 15) is further purified by the existing additives, transferred in the conjugation buffer and brought at the concentration of 0.5 mg/ml through five centrifugation cycles (15000 g x 1-3 minutes) and re-suspension in the suitable cartridges (cut-off 10 kD) provided in the Antibody Concentration & Clean-up Kit (AbSelectTM). The Ulfa-tag labelling reaction is performed at room temperature for a minimum of 3 hours by resuspending the content of a vial of the conjugation kit (Lightning-Link® Ulfa-tag Conjugation Kit) with 100 microlitres of antibody (final amount 50 micrograms) mixed in advance with 10 microlitres of "LL Modifier", and by adding at the end of incubation 10 microlitres of Quencer. The so-obtained reagent is kept at 4°C until use in the LFA test. The same conjugation procedure is also applied to an anti-Spike protein (S1) reagent having proved effectiveness (this must not be necessarily of IgA class) to act as positive control in LFA test.
3.2. **Preparation of antigen conjugated to the gold particles:** the antigen (SARS-CoV-2 (2019-nCoV) Spike S1-mFc, Sino Biological) is purified by the existing additives, transferred into the conjugation buffer and brought at the concentration of 0.5 mg/ml through five centrifugation cycles (15000 g x 1-3 minutes) and resuspension in the suitable cartridges (cut-off 10 kD) provided in the "Antibody Concentration & Clean Up Kit" (AbPureTM). The conjugation reaction covalent to the microparticles InnovaCoat® GOLD (40 nm) is performed at room temperature for 15 minutes in a volume of 50 microlitres (10 of antibody + 40 reaction buffer provided by the kit) and blocked with the addition of 5 microlitres of Quencer 10 x (still provided by the kit, InnovaCoat® GOLD (20 OD) Conjugation Kit); with the purpose of removing the not bound antigen, the reaction product (50 microlitres) is diluted with 500 microlitres of deionized water containing Quencer 1x, centrifuged (9000 g x 10 minutes) and resuspended in 50 microlitres of Quencer 1x containing BSA 0.5 % as stabilizing and blocking agent. Assuming a recovery of the microparticles of 100%, this volume should correspond to an optical density (OD) equal to 20. With the purpose of optimizing the labelling reaction three different amounts of antigen (2.5, 1 and 0.5 micrograms) were tested by keeping the above-described volumes unaltered.
3.3. **Immunochromatography for the anti SARS-CoV SP1 IgA:** the above-described conjugates can be used according to the indications contained in the Universal Lateral Flow Assay kit (Universal Lateral Flor Assay Kit, Expedeon). The capture antibody (conjugated with Ulfa-tag) is diluted at a concentration of 100 ug/ml (range 50-150 ug/ml) in running buffer + BSA 0.1% (1 mg/ml) and 20-OD antigen (see above) at 6 OD in the same buffer. 75 microlitres of buffer (in our case salivary fluid or concentrated salivary fluid) are mixed with 5 microlitres of each one of the two reagents (capture antibody and antigen/detector) and with 5 microlitres of biotinylated GOLD particles (1 OD), still provided by the kit, in a 96-well plate. After 5 minutes of incubation (required for the formation of the antigen-antibody complexes) in the well, the "universal" small strip is inserted, by dipping it from the thinnest side (sample pad). After twenty minutes of incubation at room temperature, in case of positive result, two coloured bands are observed, one at the capture line (the anti-Ulfa immobilized antibody recognizes the CoV-2SP1-GOLD + IgA anti-SP1 + anti IgA-Ulfa-tag complex) and one at the control line a (the GOLD-biotin particles are recognized by Streptavidin) (Figure 4a). The colouration absence on the capture line, even in presence of a visible control line, is interpreted as negative result. In order to verify the system operation, the salivary sample and the capture reagent (anti IgA-Ulfa-tag) can be replaced by the anti-Spike protein antibody previously conjugated with Ulfa-tag (see above); this will directly act as bridge between the beads conjugated with antigen and anti Ulfa-tag antibody on the capture line (figure 4a, right portion).

According to a different embodiment (Figure 4b):
- The capture antibody (anti IgA) is not labelled but can be directly immobilized (in form of line or spot) on the solid support;
- The anti-mouse IgG-Fc antibody, capable of recognizing directly the tail Fc present in the SARS-CoV-2 (2019-nCoV) Spike S1-mFc antigen, can be immobilized on the control line;
- The CoV-2 SP1/GOLD conjugate can be placed directly, in dried form, in a segment of the small strip upstream of the capture line ("conjugate pad"); alternatively, in order to simplify the assembly of the small strip itself, the conjugate can be placed directly (in liquid or dried form) onto the bottom of the beaker in which the proband introduces the salivary fluid after having recovered it from the concentrator. After having mixed the container content with a plastic stick and having waited for 5 minutes, the small strip can be inserted and the reaction started.

## Claims

1. A method for the diagnosis of SARS-CoV-2 infection comprising a step of detecting SARS-CoV-2 viral particles and/or anti-SARS-CoV-2 IgA-class immunoglobulins in a biological sample, wherein said biological sample is a saliva sample.

2. The method according to claim 1, wherein said step of detecting SARS-CoV-2 viral particles is carried out by means of latex beads agglutination test (LAT) and wherein said latex beads are coated with anti-SARS-CoV-2 Spike protein antibodies, in particular with anti-SARS-CoV-2 Spike protein S1-subunit antibodies.

3. The method according to claim 1, wherein said step of detecting SARS-CoV-2 viral particles is carried out by means of lateral flow immunochromatography test (Lateral Flow Assay, LFA) and comprises the steps of:
i. contacting a volume of a solution comprising said saliva sample to be analysed with a solid support; said solid support is **characterized by** (a) a first "capture" area comprising an immobilized capture agent, capable of forming a complex with one or more virus SARS-CoV-2 antigens and by (b) a second "control" area comprising an immobilized control agent capable of forming a complex with a detection agent;
ii. diagnosing SARS-CoV-2 infections when a signal visible to the naked eye appears in the first "capture" area and a signal visible to the naked eye appears in the second "control" area;
said lateral flow immunochromatography test is **characterized in that** i) a solid support comprising a membrane and/or paper is used in said step through which the solution comprising said saliva sample to be analysed migrates by capillary action; wherein said detection agent is an anti-SARS-CoV-2 antibody, said antibody is conjugated to a marker agent, in particular wherein said marker agent is a gold nanoparticle with a diameter between 10 and 80 nm; wherein said immobilized capture agent is an anti-SARS-CoV-2 Spike protein antibody, in particular said immobilized capture agent is an anti-SARS-CoV-2 Spike protein S1-subunit antibody; and wherein said control agent is an antibody capable of recognizing the Fc portion of said detection antibody.

4. The method according to any one of claims 1 to 3, wherein said detection of anti-SARS-CoV-2 IgA-class immunoglobulins is carried out by means of latex beads agglutination test (Latex Agglutination Test, LAT), wherein said latex beads are coated with native or recombinant SARS-CoV-2 virus antigens, in particular, said latex beads are coated with S1 subunits of SARS-CoV-2 Spike proteins, said S1 subunits are fused with the Fc portion of the mouse IgG1 immunoglobulin.

5. The method according to any one of claims 1 to 4, wherein a first volume of reagent comprising said latex beads is put in contact with a volume of said biological sample to be analysed, and a second volume of reagent comprising said latex beads is put in contact with a volume of a control sample for a period of time between 2 and 5 minutes, and wherein the appearance of agglutination in said saliva sample to be analysed and the absence of agglutination in said control sample indicate the presence of anti-SARS-CoV-2 IgA-class immunoglobulins in said saliva sample to be analysed.

6. The method according to any one of claims 1 to 5, wherein said step of detecting anti-SARS-CoV-2 IgA-class immunoglobulins is carried out by means of lateral flow immunochromatography test (Lateral Flow Assay, LFA), and comprises the steps of:
i. contacting a volume of a solution comprising said saliva sample to be analysed with a solid support; said solid support is **characterized by** (a) a first "capture" area comprising an immobilized capture agent, capable of forming a complex with said anti-SARS-CoV-2 IgA-class immunoglobulins, and (b) a second "control" area comprising an immobilized control agent capable of forming a complex with a detection agent;
ii. diagnosing the presence of salivary anti-SARS-CoV-2 antibodies when a signal visible to the naked eye appears in the first "capture" area and a signal visible to the naked eye appears in the second "control" area;
said lateral flow immunochromatography test is **characterized in that** a solid support i) comprising a membrane and/or paper is used in said step through which the solution comprising said saliva sample to be analysed migrates by capillary action; wherein said detection agent is an agent capable of binding said anti-SARS-CoV-2 IgA-class immunoglobulins, in particular wherein said detection agent is the S1 subunit of SARS-CoV-2 Spike proteins, fused with the Fc portion of the mouse IgG1 immunoglobulin, said S1 subunit is conjugated to a marker agent; wherein said marker agent is a gold nanoparticle with a diameter between 10 and 80 nm; and wherein said immobilized capture agent is an anti-lgA-class immunoglobulin antibody.

7. The method according to any one of claims 1 to 6, comprising a concentration step of said biological sample preceding said detection step, wherein said concentration step is carried out by means of direct osmosis.

8. The method for the evaluation of the immune response to SARS-CoV-2 virus comprising the steps of a method according to any one of claims 4 to 7.

9. A kit for the diagnosis of SARS-CoV-2 infection comprising (i) one or more means for the detection, by agglutination tests, of SARS-CoV-2 viral particles in a saliva sample, and (ii) one or more solid supports for agglutination tests, wherein said one or more means for the detection by agglutination tests comprise agglutination means coated with a reagent capable of binding one or more antigens of SARS-CoV-2 virus, wherein said reagent capable of binding one or more antigens of SARS-CoV-2 virus is an anti-SARS-CoV-2 Spike protein antibody, in particular it is an anti-SARS-CoV-2 Spike protein S1-subunit antibody, or an anti-SARS-CoV-2 Spike protein S2-subunit antibody; and wherein said agglutination means are latex beads.

10. The kit for the diagnosis of SARS-CoV-2 infection comprising (i) one or more means for the detection, by means of lateral flow immunochromatography test (LFA), of SARS-CoV-2 viral particles in a saliva sample, and (ii) one or more aliquots of a detection agent, wherein said one or more means for the detection by means of lateral flow immunochromatography test (LFA) are solid support means capable of absorbing said saliva sample by capillary action; said solid support means are **characterized by** (a) a first "capture" area comprising an immobilized capture agent, capable of forming a complex with one or more SARS-CoV-2 virus antigens and (b) a second "control" area comprising an immobilized control agent capable of forming a complex with a detection agent;
wherein said detection agent is an anti-SARS-CoV-2 antibody, said antibody is conjugated to a marker agent, in particular wherein said marker agent is a gold nanoparticle with a diameter between 10 and 80 nm;
wherein said immobilized capture agent is an anti-SARS-CoV-2 Spike protein antibody, in particular said immobilized capture agent is an anti-SARS-CoV-2 Spike protein S1-subunit antibody;
and wherein said immobilized control agent is an antibody capable of recognizing the Fc portion of said detection antibody.

11. The kit for the detection of mucosal immunity to SARS-CoV-2 comprising (i) one or more means for the detection, by agglutination tests, of anti-SARS-CoV-2 IgA-class immunoglobulins in a saliva sample, and (ii) one or more solid supports for agglutination tests, wherein said one or more means for the detection by agglutination tests are agglutination means coated with a reagent capable of binding said anti-SARS-CoV-2 IgA-class immunoglobulins, wherein said reagent capable of binding said anti-SARS-CoV-2 IgA-class immunoglobulins is constituted by native or recombinant SARS-CoV-2 virus antigens, in particular it is the S1 subunit of SARS-CoV-2 Spike proteins, fused with the Fc portion of the mouse IgG1 immunoglobulin; and wherein said agglutination means are latex beads.

12. The kit for the detection of mucosal immunity to SARS-CoV-2 comprising (i) one or more means for the detection, by means of lateral flow immunochromatography test (LFA), of anti-SARS-CoV-2 IgA-class immunoglobulins in a saliva sample, and (ii) one or more aliquots of a detection agent, wherein said one or more means for the detection by means of lateral flow immunochromatography test (LFA) are solid support means capable of absorbing said saliva sample by capillary action; said solid support means are **characterized by** (a) a first "capture" area comprising an immobilized capture agent, capable of forming a complex with said anti-SARS-CoV-2 IgA-class immunoglobulins and (b) a second "control" area comprising an immobilized control agent capable of forming a complex with a detection agent;
wherein said detection agent is capable of binding said anti-SARS-CoV-2 IgA-class immunoglobulins, in particular wherein said detection agent is the S1 subunit of SARS-CoV-2 Spike proteins, fused with the Fc portion of the mouse IgG1 immunoglobulin, said S1 subunit is conjugated to a marker agent; wherein said marker agent is a gold nanoparticle with a diameter between 10 e 80 nm;
wherein said immobilized capture agent is an anti-lgA-class immunoglobulin antibody.

13. The kit according to claim 12, further comprising (iii) one or more aliquots of anti-lgA-class immunoglobulin antibodies labelled with Ulfa-tag capable of forming a complex with said IgA-class immunoglobulins, and (iv) one or more means for the detection, by means of lateral flow immunochromatography test (LFA), of anti-SARS-CoV-2 IgA-class immunoglobulins in a saliva sample wherein said immobilized capture agent is an anti-Ulfa-tag antibody; said immobilized control agent is streptavidin; said detection agent comprises a first detection agent consisting of biotin-labelled gold nanoparticles capable of binding said immobilized control agent, and a second detection agent consisting of the S1 subunit of SARS-CoV-2 Spike proteins fused with the Fc portion of mouse IgG1 immunoglobulin, conjugated to a 40-nm gold nanoparticle, wherein said S1 subunit is capable of forming a complex with said IgA-class immunoglobulins.

14. The kit combination comprising at least a kit according to any one of claims 9 or 10 and at least a kit according to any one of claims 11 to 14 for diagnosing SARS-CoV-2 infection, and/or for evaluating the immune system response to SARS-CoV-2 virus.

15. The kit according to any one of claims da 9 to 15, further comprising a device for concentrating said saliva sample, wherein said device includes a dialysis membrane with a cut-off equal to 10 kDa.
